# EUROPEAN PATENT SPECIFICATION

(11) **EP 0 492 594 B1**
(45) Date of publication and mention of the grant of the patent: **20.09.1995**
(21) Application number: 91122166.1
(22) Date of filing: 23.12.1991
(51) Int. Cl.: C07C 25/125, C07C 17/12

(54) **Bromination of ortho-xylene**
Verfahren zur Bromierung von Ortho-Xylol
Procédé pour la bromation d'ortho-xylène

(30) Priority: 26.12.1990 IL 96792
(43) Date of publication of application: 01.07.1992
(73) Proprietor: Bromine Compounds Ltd., Beer-Sheva 84101 (IL)
(72) Inventor: Oren, Jakob, Qiryat Bialik (IL); Hermolin, Joshua, Ramat Hasharon 47445 (IL)
(74) Representative: Perani, Aurelio

(56) References cited:
- WO-A-91/13047
- US-A- 2 591 498
- US-A- 2 659 760
- US-A- 3 012 035
- 'ORGANIC SYNTHESIS COLLECTIVE VOLUME 3' 1955

## Description

### Field of the Invention

The present invention relates to a process for the preparations of dimethyl-bromobenzene having a high content of 3,4-dimethyl-bromobenzene isomer. More particularly, the invention relates to a process for the bromination of ortho-xylene at temperatures below 0°C.

### BACKGROUND OF THE INVENTION

3,4-Dimethyl-bromobenzene is an important intermediate for the preparation of a variety of chemical compounds, such as precursors of aromatic bis (therephtalic anhydride) compounds [U.S. Patent No. 4,600,798.]. The properties of the high-performance, polymers prepared from such intermediates are very much dependent, on the purity of the components used. For example, over-bromination in the preparations of 3,4-dimethyl-bromobenzene would lead to cross-linking of the final polymer and unsupportable rigidity. The lack of isomeric integrity would also affect the physical and mechanical properties of the final products. The difficulty in achieving such high levels of purity is clear from the consideration of the boiling point of the 3,4-dimethyl-bromobenzene isomer (215°C), and that of the 2,3-isomer (214°C).

### The Prior Art

The bromination of ortho-xylene has been extensively investigated in the art, and the selectivity of the bromination reaction toward the formation of highly pure 3,4-dimethyl-bromobenzene has been addressed, however, often only with respect to the dibromo derivative. The effect of the temperature, (in the range between -5°C and +40°C) on the ratio of the 3,4- and 2,3-isomers has been mentioned but the art has so far failed to provide an industrially applicable method by means of which a highly selective reaction can be carried out, to provide a final product with a high content of the 3,4-isomer.

Ortho-xylene has been brominated, in the presence of Fe catalysts [W.A. Wisansky and S. Ansbacher, Organic Synthesis, Collective Vol. No. 3, p. 138-140 (1955)] . This reference also indicates that I₂ has been previously used, as a catalyst The authors also report that it has been recommended to improve the purity of such products by sulfonation and recrystallization, again demonstrating the difficulty in achieving a product of high purity. Another publication [A.A. Shereshevski and V.M. Berezovskii; Kim. Farm. Zh., 3(10), 52-53 (1969)] reports the formation of 3,4-dimethyl-bromobenzene in yields higher than 80%, and the best results were reportedly obtained at temperatures of 30-40°C. It is of interest to note that although these authors worked at a temperature range, from 0-40°C, they chose to operate at the higher range of temperatures. However, this reference does not address the content of the other possible isomer, viz. 2,3-dimethyl-bromobenzene, in the product. An additional citation [F.B. Naidis, et al. Khim. Farm. Zh., 8(1), 31-34-(1974)] compares Fe and I₂ as catalysts in the bromination of ortho-xylene, but again does not differentiate between the 3,4- and the 2,3- isomers. In a later report, infra-red and chromatographic analyses were used to determine the composition vis-a-vis these isomers in the bromination product mixtures [I. Meirovics, et al. Latv. PSR Zinat. Akad. Vestis Khim. Ser., 1970 (5), 591-594 (1970), and V.S. Tarnavskii et al., Khim. Farm. Zh., 11(2), 92-93(1977)]. The isomer ratios found were in the range of 75-82% of 3,4-dimethyl-bromobenzene to 25-18% of 2,3-dimethyl-bromobenzene.

Bromination in the presence of AlCl₃ or ZnO [SU 544,647] led only to a decrease in the production of dibromo-xylenes. The molar ratio of the two isomers is similar to that of previous publications.

Z.I. Krutikova & I.G. Skorina, [Khim-Farm. Zh. 1977, 11 (2), 92-93], studied the xylene bromination reaction over a temperature range of -5°C to +40°C using a special catalyst. They report no significant difference in the results over this temperature range. However, from the data given it appears that their ability to detect minor but significant differences was limited.

In a series of three papers the bromination of ortho-xylene was conducted using Br₂/ H₂O₂ [SU 891,619], HBr/NaOCl [SU 1,097,592] and HBr/H₂O₂ [SU 636,217 and M.S. Salakhov et al., Zh. Prikl. Khim., 1983, 56(4),840-843]. In these references, again, the isomer ratios of the monobromo-xylenes is similar to that previously reported. These authors surprisingly reported that it was preferable to operate at the higher end of the temperature range, 20°-40°C. Their thermodynamic data support this conclusion. However, had they extended their studies to temperatures lower than 10°C below zero, different results would have been obtained.

Bromination of ortho-xylene has also been attempted in various polar solvents, such as acetonitrile, nitromethane and nitrobenzene in the temperature range between 0°C and 40°C. While some solvent effects have been found on the relevant isomeric ratio [A. Persoons, et al. Bull. Soc. Chim. Fr. (7), 2729-2736 (1968)], the results were not substantially different from those previously obtained in the art. From the cited work it is clear that one cannot predict the ratio of the 3,4-isomer to the 2,3-isomer simply on the basis of temperature, but that the nature of the reaction medium is an important factor.

The highest molar ratio of 3,4-dimethyl-bromobenzene to 2,3-dimethyl-bromobenzene reported in the art was obtained in SO₂ [J.B. Canselier; Bull. Soc. Chim. Fr. (2), 762-764 (1972)]. Operation in the presence of trifluoroacetic acid is also reported, but the isomeric ratio is not as good as that obtained in SO₂. However, operating with SO₂, which is volatile and poisonous, causes severe contamination of the evolving HBr and of the environment, and is therefore not easily industrially applicable.
A regioselective process for preparing dimethylbromobenzene with a high ratio of the 3,4-dimethylbromobenzene to the 2,3-dimethylbromobenzene isomer is disclosed in patent application W091/13047, published on 05.09.1991 (Art. 54(3) Field). Such a result is obtained by conducting the bromination of xylene either in the darkness in the absence of solvents or in a liquid sulfur dioxide medium, at a temperature of -20°C to 40°C.
It is therefore clear that the art has so far failed to provide a simple and economic process by means of which mono-bromoxylenes can be obtained with a high selectivity toward the 3,4-dimethyl-bromobenzene isomer, which is industrially applicable, and which does not lead to unwanted contamination of the products.

### SUMMARY OF THE INVENTION

It has now been surprisingly found, and this is an object of the present invention, that ortho-xylene can ben reacted with bromine at a low temperature to produce 3,4-dimethyl-bromobenzene in high selectivity. It has further been found, and this is another object of the invention, that contrary to what was commonly believed, reaction at low temperatures is industrially feasible, and does not result in excessively long reaction times.

The process for the preparation of dimethyl-bromobenzene with a high ratio of 3,4-dimethyl-bromobenzene to the 2,3-dimethyl-bromobenzene isomer comprises reacting ortho-xylene with Br₂ in darkness in the presence of a catalyst at a reaction temperature between -10°C and -70⁰C. The reaction can be carried out without solvents, or it can be effected in a suitable solvent. Suitable solvents will be easily identified by the skilled chemist, as they are required to be substantially unreactive under the reaction conditions, and to have a melting point which is sufficiently below the bromination temperature to permit the reaction to take place. Preferred solvents of this type are suitable saturated aliphatic hydrocarbons and aliphatic and aromatic halogenated hydrocarbons.

When it is desired to carry out the reaction in the substantial absence of a solvent, the preferred temperature range will be between -10°C and -30°C.

In order for the reaction to proceed at a sufficient speed, it is necessary to employ a suitable catalyst. Such catalysts are known m the art, and a stable catalyst can be selected by the skilled person. Preferred catalysts for this purpose are selected from the group consisting essentially of I₂, Fe, FeBr₃, FeCl₃, Al, AlBr₃, AlCl₃ and their mixtures. Further encompassed by the present invention are dimethyl-bromobenzene mixtures having a high content of the 3,4-dimethyl-bromobenzene isomer, whenever these mixtures are produced by the process of the present invention.

### Brief Description of the Drawings

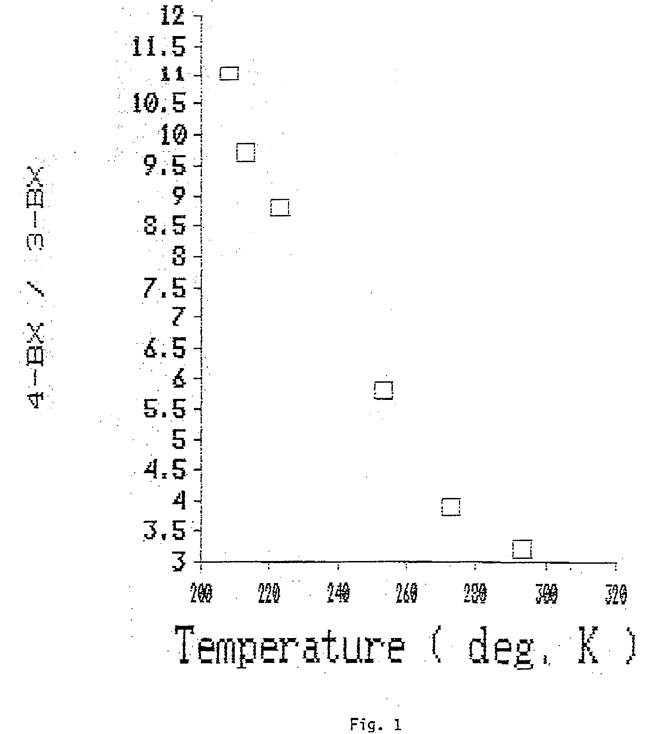

- Fig. 1 illustrates the molar ratio 3,4-dimethyl-bromobenzene :
   2,3-dimethyl-bromobenzene, as a function of reaction temperature.

All the above and other characteristics and advantages of the process of the invention will be further understood by the following non-limitative description of examples thereof.

### Example 1

### Bromination of o-xylene in dichloromethane (DCM)

Bromine (152 g, 0.95 moles) was added to a stirred solution of o-xylene (106 g, 1.0 mole) and FeBr₃ (2.96 g, 0.01 mole) in dichloromethane (200 ml) in complete darkness in a four-necked round-bottomed flask equipped with a stirrer, a condenser, a dropping funnel and a thermometer. To the top of the condenser there was attached a trap to absorb HBr released during the reaction. The addition of the Br₂ took place over one hour at the temperature T₁ of Table I below, after which the reaction mixture was stirred in the dark at the same temperature for a further t hours. The progress of the reaction and its completion were followed by means of GC analysis. After the conversion of 90% of the o-xylene the reaction was terminated by increasing the temperature to 0°C, at which temperature the bromine reacted entirely and most of the HBr was evaporated from the product mixture. Then water was added to the product mixture with stirring. The Fe compound catalyst and the water were separated, and the organic phase was analyzed for o-xylene, 3,4-dimethyl-bromobenzene, 2,3-dimethyl-bromobenzene and dibromoxylenes as follows :

### GC Analysis

Gas-chromatograph HP 5890
Oven: Initial temp. 50°C, held three minutes, then raised to 220°C at 7°C/min.
Injector: 200°C
Detector: 250°C
Column: Stabilwax (Carbowax 20M), 30 m x 0.25 mm ID, 0.25 µm film thickness
Split ratio: 1:70
Injection amounts: 1 µl
Flow: 0.6 ml/min.

### Retention time, min.:

o-xylene: 9.16
2,3-dimethyl-bromobenzene: 18.35
3,4-dimethyl-bromobenzene: 18.70
dibromoxylenes (three isomers): 25.34; 26.64; 26.82
The results are given in Table I and attached Fig. 1.

**Table I**

| Reaction Temperature T₁ °C | 4-BX/3-BX Molar Ratio | BX Yield % | DBX Yield % | Reaction Time t hours |
|---|---|---|---|---|
| 20 | 3.2 | 95.5 | 4.8 | <0.5 |
| 0 | 3.9 | 96.9 | 3.1 | <0.5 |
| -20 | 5.8 | 99.0 | 1.0 | 1.0 |
| -50 | 8.8 | 99.3 | 0.7 | 1.5 |
| -60 | 9.7 | 99.3 | 0.7 | 2.0 |
| -65 | 11.0 | 99.3 | 0.7 | 2.5 |

### Legend:

a) Reactions at T₁ ≧ -20°C are given for comparison only. In such cases, the post reactions (at 0°C) is not necessary.
b) BX -3,4-dimethyl bromobenzene and 2,3-dimethyl-bromobenzene
c) 4-BX - 3,4-dimethyl-bromobenzene
d) 3-BX - 2,3-dimethyl-bromobenzene
e) DBX -dibromoxylenes (three isomers)
f) The yields are based on reacted o-xylene.

### Example 2

Bromine (152 g, 0.95 moles) was added to stirred o-xylene (106 g, 1.0 mole) and FeBr₃ (2.96 g, 0.01 mole) in the dark in a four-necked round-bottomed flask equipped with a stirrer, a condenser, a dropping funnel and a thermometer. To the top of the condenser there was attached a trap to absorb HBr released during the reaction. The addition took place over one hour at the temperature T₁ (see Table II), after which the reaction mixture was stirred at the same temperature for a further t hours. The progress of the reaction and its completion were followed by means of GC analysis. After the conversion of 90% of the o-xylene the reaction was terminated by increasing the temperature to 0°C, at which temperature the bromine reacted entirely, and most of the HBr was evaporated from the product mixture. Then water was added to the product mixture with stirring. The Fe compound catalyst and the water were separated and the organic phase was GC analyzed for o-xylene, 3,4-dimethyl-bromobenzene, 2,3-dimethyl-bromobenzene and dibromoxylenes as mentioned in Example 1.

The results are given in Table II.

**Table II**

| Reaction Temperature T₁ °C | 4-BX/3-BX Molar Ratio | BX Yield % | DBX Yield % | Reaction Time t hours |
|---|---|---|---|---|
| 20 | 3.2 | 94.8 | 5.2 | <0.5 |
| 0 | 3.9 | 96.5 | 3.5 | <0.5 |
| -20 | 5.8 | 99.0 | 1.0 | 1.0 |
| -27 | 6.3 | 99.2 | 0.8 | 1.5 |

a) Reactions at T₁ ≧ -20°C are given far comparison only. In such cases, the post reaction (at 0°C) is not necessary.
b) BX - 3,4,dimethyl-bromobenzene and 2,3-dimethyl-bromobenzene
c) 4-BX - 3,4-dimethyl-bromobenzene
d) 3-BX - 2,3-dimethyl-bromobenzene
e) DBX - dibromoxylenes (three isomers)
f) The yields are based on reacted o-xylene.

The above description and examples have been given for the purpose of illustration, and are not intended to constitute a limitation of the invention. Many modifications can be effected in the process of the invention. For instance, different solvents and solvent mixtures can be employed, or different catalysts can be utilized, various reaction temperatures can be selected, all without exceeding the scope of the invention.

## Claims (Claims for the following Contracting State(s): BE, DE, FR, GB, IT)

1. A process for the preparation of dimethyl-bromobenzene with a high ratio of the 3,4-dimethyl-bromobenzene to the 2,3-dimethyl-bromobenzene isomer, comprising reacting o-xylene with Br₂ in complete darkness in the presence of a catalyst at a reaction temperature lower than -20°C but not lower than -70°C.

2. A process according to claim 1, wherein the bromination reaction is carried out in a suitable solvent.

3. A process according to claim 2, wherein the solvent is a saturated aliphatic hydrocarbon or an aliphatic or aromatic halogenated hydrocarbon which is substantially unreactive under the reaction conditions, the said solvent having a melting point below the bromination temperature.

4. A process according to claim 1, wherein the bromination reaction is carried out in the absence of a solvent, at a temperature not lower than -30°C.

5. A process according to any one of claims 1 to 4 wherein the catalyst is selected from the group consisting essentially of I₂, Fe, FeBr₃, FeCl₃, Al, AlBr₃, AlCl₃ and their mixtures.

6. Dimethyl-bromobenzene having a high content of the 3,4-dimethyl-bromobenzene isomer, whenever prepared by the process of any one of claims 1 to 5.

## Claims (Claims for the following Contracting State(s): ES, NL)

1. A process for the preparation of dimethyl-bromobenzene with a high ratio of the 3,4-dimethyl-bromobenzene to the 2,3-dimethyl-bromobenzene isomer, comprising reacting o-xylene with Br₂ in complete darkness in the presence of a catalyst at a reaction temperature between -10°C and -70°C.

2. A process according to claim 1, wherein the bromination reaction is carried out in a suitable solvent.

3. A process according to claim 2, wherein the solvent is a saturated aliphatic hyrocarbon or an aliphatic or aromatic halogenated hycrocarbon which is substantially unreactive under the reaction conditions, the said solvent having a melting point below the bromination temperature.

4. A process according to claim 1, wherein the bromination reaction is carried out in the absence of a solvent, at a temperature in the range between -10°C and -30°C.

5. A process according to any one of claims 1 to 4, wherein the catalyst is selected from the group consisting essentially of I₂, Fe, FeBr₃, FeCl₃, Al, AlBr₃, AlCl₃ and their mixtures.

6. Dimethyl-bromobenzene having a high content of the 3,4-dimethyl-bromobenzene isomer, whenever prepared by the process of any one of claims 1 to 5.

## Patentansprüche (Patentansprüche für folgende(n) Vertragsstaat(en): BE, DE, FR, GB, IT)

1. Verfahren zur Herstellung von Dimethylbrombenzol mit einem hohen Verhältnis zwischen dem 3,4-Dimethylbrombenzol-Isomer und dem 2,3-Dimethylbromoenzol-Isomer, das umfaßt die Umsetzung von o-Xylol mit Br₂ in vollständiger Dunkelheit in Gegenwart eines Katalysators bei einer Reaktionstemperatur unter -20°C, jedoch nicht unter -70°C.

2. Verfahren nach Anspruch 1, wobei die Bromierungsreaktion in einem geeigneten Lösungsmittel durchgeführt wird.

3. Verfahren nach Anspruch 2, worin das Lösungsmittel ein gesättigter aliphatischer Kohlenwasserstoff oder ein aliphatischer oder aromatischer halogenierter Kohlenwasserstoff ist, der unter den Reaktionsbedingungen im wesentlichen nicht-reaktionsfähig ist, wobei das genannte Lösungsmittel einen Schmelzpunkt unterhalb der Bromierungstemperatur aufweist.

4. Verfahren nach Anspruch 1, wobei die Bromierungsreaktion in Abwesenheit eines Lösungsmittels bei einer Temperatur nicht unter -30°C durchgeführt wird.

5. Verfahren nach einem der Ansprüche 1 bis 4, worin der Katalysator ausgewählt wird aus der Gruppe, die besteht im wesentlichen aus J₂, Fe, FeBr₃, FeCl₃, Al, AlBr₃, AlCl₃ und Mischungen davon.

6. Dimethylbrombenzol mit einem hohen Gehalt an dem 3,4-Dimethylbrombenzol-Isomer, hergestellt nach dem Verfahren nach einem der Ansprüche 1 bis 5.

## Patentansprüche (Patentansprüche für folgende(n) Vertragsstaat(en): ES, NL)

1. Verfahren zur Herstellung von Dimethylbrombenzol mit einem hohen Verhältnis zwischen dem 3,4-Dimethylbrombenzol-Isomer und dem 2,3-Dimethylbrombenzol-Isomer, das umfaßt die Umsetzung von o-Xylol mit Br₂ in vollständiger Dunkelheit in Gegenwart eines Katalysators bei einer Reaktionstemperatur zwischen -10 und -70°C.

2. Verfahren nach Anspruch 1, bei dem die Bromierungsreaktion in einem geeigneten Lösungsmittel durchgeführt wird.

3. Verfahren nach Anspruch 2, wobei das Lösungsmittel ein gesättigter aliphatischer Kohlenwasserstoff oder ein aliphatischer oder aromatischer halogenierter Kohlenwasserstoff ist, der unter den Reaktionsbedingungen im wesentlichen nicht-reaktionsfähig ist, wobei das genannte Lösungsmittel einen Schmelzpunkt unterhalb der Bromierungstemperatur aufweist.

4. Verfahren nach Anspruch 1, bei dem die Bromierungsreaktion in Abwesenheit eines Lösungsmittels bei einer Temperatur in dem Bereich zwischen -10 und -30°C durchgeführt wird.

5. Verfahren nach einem der Ansprüche 1 bis 4, worin der Katalysator ausgewählt wird aus der Gruppe, die im wesentlichen besteht aus J₂, Fe, FeBr₃, FeCl₃, Al, AlBr₃, AlCl₃ und Mischungen davon.

6. Dimethylbrombenzol, das einen hoben Gehalt an dem 3,4-Dimethylbrombenzol-Isomer aufweist, hergestellt nach dem verfahren nach einem der Ansprüche 1 bis 5.

## Revendications (Revendications pour l'(les) Etat(s) contractant(s) suivant(s): BE, DE, FR, GB, IT)

1. Procédé de préparation de diméthylbromobenzène avec un rapport élevé de l'isomère 3,4-diméthylbromobenzène à l'isomère 2,3-diméthylbromobenzène, comprenant la réaction de o-xylène avec Br₂ à L'obscurité totale en présence d'un catalyseur à une température réactionnelle inférieure à -20°C mais non inférieure à -70°C.

2. Procédé suivant la revendication 1, dans lequel la réaction de bromation est conduite dans un solvant convenable.

3. Procédé suivant la revendication 2, dans lequel le solvant est un hydrocarbure aliphatique saturé ou un hydrocarbure aliphatique ou aromatique halogéné qui est pratiquement non réactif dans les conditions de la réaction, ledit solvant ayant un point de fusion inférieur à la température de bromation.

4. Procédé suivant la revendication 1, dans lequel la réaction de bromation est conduite en l'absence d'un solvant, à une température non inférieure à -30°C.

5. Procédé suivant l'une quelconque des revendications 1 à 4, dans lequel le catalyseur est choisi dans le groupe consistant essentiellement en I₂, Fe, FeBr₃, FeCl₃, Al, AlBr₃, AlCl₃ et leurs mélanges.

6. Diméthylbromobenzène ayant une haute teneur en l'isomère 3,4-diméthylbromobenzène, préparé par le procédé suivant l'une quelconque des revendications 1 à 5.

## Revendications (Revendications pour l'(les) Etat(s) contractant(s) suivant(s): ES, NL)

1. Procédé de préparation de diméthylbromobenzène avec un rapport élevé de l'isomère 3,4-diméthylbromobenzène à l'isomère 2,3-diméthylbromobenzène, comprenant la réaction de o-xylène avec Br₂ à l'obscurité totale en présence d'un catalyseur à une température réactionnelle de -10°C à -70°C.

2. Procédé suivant la revendication 1, dans lequel la réaction de bromation est conduite dans un solvant convenable.

3. Procédé suivant la revendication 2, dans lequel le solvant est un hydrocarbure aliphatique saturé ou un hydrocarbure aliphatique ou aromatique halogéné, qui est pratiquement non réactif dans les conditions de la réaction, ledit solvant ayant un point de fusion inférieur à la température de bromation.

4. Procédé suivant la revendication 1, dans lequel la réaction de bromation est conduite en l'absence de solvant, à une température comprise dans l'intervalle de -10°C à -30°C.

5. Procédé suivant l'une quelconque des revendications 1 à 4, dans lequel le catalyseur est choisi dans le groupe consistant essentiellement en I₂, Fe, FeBr₃, FeeCl₃, Al, AlBr₃, AlCl₃ et leurs mélanges.

6. Diméthylbromobenzène ayant une haute teneur en l'isomère 3,4-diméthylbromobenzène, préparé par le procédé suivant l'une quelconque des revendications 1 à 5.
